# EUROPÄISCHE PATENTSCHRIFT

(11) **EP 0 859 554 B1**
(45) Veröffentlichungstag und Bekanntmachung des Hinweises auf die Patenterteilung: **22.05.2002**
(21) Anmeldenummer: 96933306.1
(22) Anmeldetag: 23.10.1996
(51) Int. Cl.: A23L 1/22, A23P 1/02, A61K 7/46

(54) **AROMENGRANULAT**
AROMATIC GRANULATED MATERIAL
GRANULES D'AROMES

(30) Priorität: 27.10.1995 CH 303795; 15.10.1996 CH 251896
(43) Veröffentlichungstag der Anmeldung: 26.08.1998
(73) Patentinhaber: Givaudan SA, 1214 Vernier-Genève (CH)
(72) Erfinder: MENZI, Heini, CH-8625 Gossau (CH); PERREN, Matthias, CH-5200 Brugg (CH); RINGGENBERG, Rudolf, CH-8153 Rümlang (CH)
(74) Vertreter: Patentanwälte Schaad, Balass, Menzl & Partner AG
(86) Internationale Anmeldenummer: CH9600373
(87) Internationale Veröffentlichungsnummer: WO9716078

(56) Entgegenhaltungen:
- EP-A- 0 011 324
- EP-A- 0 070 719
- WO-A-91/09989
- WO-A-95/15821
- WO-A-96/23423

## Beschreibung

Die vorliegende Erfindung betrifft ein Verfahren zur Herstellung von sphärischen bzw. im wesentlichen sphärischen, praktisch staubfreien, freifliessenden, mechanisch stabilen Aromen- und Riechstoffgranulaten mit enger Korngrössenverteilung.

Unter "sphärisch" wird im vorliegenden Fall ein bei visueller Betrachtung, mit unbewaffnetem Auge, kugelförmiges, bzw. ein im wesentlichen kugelförmiges Material verstanden.

Aromengranulate sind bekannt. EP 0070719 beschreibt die Herstellung von Aromengranulaten, die in einem konventionellen Fliessbett, gebildet durch reine Aufwirbelung von Luft, hergestellt werden. Diese Produkte weisen jedoch die Nachteile einer ungenügenden mechanischen Festigkeit, des relativ grossen Staubanteils und einer beschränkten Fliessfähigkeit auf.

Ziel der vorliegenden Erfindung war ein Verfahren, das erlaubt, Produkte herzustellen, die die geschilderten Nachteile nicht mehr aufweisen, und dementsprechend bei ihrer Verwendung, also bei der Einarbeitung in das zu aromatisierende bzw. parfümierende Material, eindeutig bevorzugt werden. Im Vordergrund stehen Nahrungsmittel, Genussmittel und Getränke, Pharmazeutika, kosmetische Produkte, Hygieneprodukte, z.B. Mundhygieneprodukte, Windeln, Seifen, Detergenzien, Haushaltprodukte, etc.

Zusätzlich können die erfindungsgemäss erhältlichen Aromen- und Riechstoffgranulate in einem zweiten Schritt auf einfache Weise mit einer Umhüllung versehen werden, ohne dass der nun erfindungsgemäss verwendete Rotorgranulator ausgeschaltet oder umgerüstet, oder Produkt transferiert werden muss. Die Umhüllung bezweckt auch eine verbesserte Einkapselung der Wirkstoffe, eine Aenderung, d.h. Verbesserung des Löslichkeitsverhaltens oder eine gezielte Schutzwirkung zu erreichen.

Rotorgranulatoren wurden bis anhin in der Produktion von Waschmittelzusätzen, Düngemitteln und Pharmawirkstoffen eingesetzt (siehe z.B. den Firmenprospekt GRCG Typ 1-200 der Firma Glatt, Pratteln, Schweiz Juni 1992]).

Die Apparatur besteht im wesentlichen aus einer Fliessbetteinrichtung und der Zu- und Ablufteinheit. Zweckmässig ist z.B. ein zylindrischer Behälter mit vertikaler Drehachse, dessen Boden um die Zentralachse rotiert. Der Zylindermantel ist zweckmässigerweise feststehend. Die Drehzahl beträgt zweckmässigerweise ca. 50-500/min. Das im Prozess vorgelegte Kernmaterial wird mittels einer rotierenden Bodenplatte in Bewegung gesetzt. Durch den (peripheren) Ringspalt zwischen rotierender Bodenplatte und feststehender Behälterwand strömt eine relativ geringe Menge Luft, die zusammen mit der Rotationsbewegung der Bodenplatte das vorgelegte Gut in Bewegung versetzt, "fluidisiert". Im Vergleich zu früheren Technologien, die die nötige Bewegung des Gutes nur mit Hilfe der Luft erzeugten, wird nur ca. 1/5 - ca. 1/3 der Luft benötigt, um den vorgelegten Feststoff zu fluidisieren, zu bewegen.

Das neue Verfahren ist dadurch gekennzeichnet, dass in ein in einem Fliessbett-Rotorgranulator fluidisierten Kernmaterial eine Aroma- oder Riechstoffemulsion submers - in das Fliessbett - eingesprüht und so granuliert wird. Das Verfahren beinhaltet also im wesentlichen die Verwendung eines Fliessbett-rotorgranulators bei der Herstellung von sphärischen Aromen- und Riechstoffgranulaten.

Gegen die Verwendung solcher Apparaturen für Aromen- bzw. Riechstoffkompositionen sprach der doch immer noch relativ hohe Luftdurchsatz und die doch relativ lange Reaktionszeit - dies im Hinblick auf zu befürchtende Verluste an - insbesondere - leicht flüchtigen Komponenten.

Das Kernmaterial ist ein für die Herstellung von Aromen- oder Riechstoffgranulat üblicherweise verwendeter, in der Industrie zugelassener oder pharmazeutisch anwendbarer Feststoff, zweckmässigerweise mit Korngrössen von ca. 0,02 bis ca. 3,0 mm, insbesondere von 0,2 bis ca. 1,5 mm Durchmesser. Es ist beispielsweise ein Kohlenhydrat, z.B. ein Zucker, wie Glucose, Lactose, Saccharose, oder auch ein Produkt komplexerer Zusammensetzung, wie Fruchtpulver, z.B. Orangensaftpulver, oder Gemüsepulver, z.B. Karottensaftpulver, oder ein Zuckeralkohol, wie Isomalt, oder Pectin, hydrolysiertes Pflanzenprotein (HVP), Nahrungsfasern, z.B. Trester, Weizenfasern, Cellulosefasern, etc., oder ein organisches oder anorganisches Salz, z.B. ein Citronensäuresalz oder Kochsalz, aber auch Kräuterpulver, Gewürzpulver, Teepulver kommen in Frage.

Das Trägermaterial für die eingesprühte Emulsion wird generell aus für diese Zwecke üblichen Materialien ausgewählt, es ist zweckmässigerweise ein Kohlenhydrat, z.B. chemisch modifizierte Stärke, abgebaute Stärke (Dextrin, Maltodextrin); natürliche Harze, Exudate, z.B. Gummi arabicum, Pflanzenextrakte, wie Carragenan, Alginate, etc. ein Protein, z.B. ein Milchprotein, oder Gelatine, etc. oder eine Kombination solcher Stoffe.

Als Lösungsmittel für die Emulsion können beispielsweise Wasser oder Wasser/Ethanolgemische dienen.

Bei der Granulierung können auch übliche Zusatzstoffe, wie künstliche Süssstoffe, Lebensmittelfarbstoffe, Vitamine, Antioxidantien, Antischaummittel, Kohlensäuregeneratoren wie Weinsäure, Genusssäuren wie Citronensäure, oder zusätzliche Geschmackstoffe, etc. verwendet werden, die dem Kernmaterial oder der Sprühemulsion zugesetzt werden können.

Nach der Granulierung kann eine Umhüllung der Partikel erfolgen, z.B. durch Aufsprühen, z.B. einer Lösung, Emulsion oder Schmelze einer für diese Zwecke bekanntermassen geeigneten, eine Schutzhaut bzw. einen Film bildenden Substanz bzw. eines Substanzgemisches, wie z.B. Fett, oder modifizierte Cellulose, Gelatine, Pflanzen- oder Tierextrakte, Exudate, z.B. Gummi arabicum, abgebaute Stärke oder chemisch modifizierte Stärke, pharmazeutisch anwendbare Kunststoffe, z.B. Polyvinylpyrrolidon, Polyäthylenglykol, etc.

Die geeigneten Lufttemperaturen sind erhöhte Temperaturen, z.B. ca. 30°, bzw. 40° - ca. 80°C, bevorzugt ca. 40°, bzw. 50 - ca. 70°C.

Es kommen alle gängigen Aromen und Riechstoffe in Frage, also z.B. Richtung Fleisch, Käse, Frucht, z.B. Citrus, Beeren, Tabak, Blumen, Holz, Gewürz, Ambra, etc., die in der Industrie Verwendung finden können. Als Aromen- und Riechstoffkomponenten kommen alle die bisher für Aromen und Riechstoffe(granulate) üblicherweise verwendeten Komponenten in Frage, also einzelne solche Komponenten, z.B. Menthol oder Vanillin, etc. - oder aber ätherische Oele oder Fraktionen oder Gemische von Aroma- und Riechstoffkompositionen. Die einzelnen Komponenten können natürlichen (pflanzlichen oder tierischen) oder synthetischen Ursprungs sein.

Die enge Korngrössenverteilung wird im wesentlichen erzielt durch das Zusammenwirken der Parameter: Korngrösse des Trägermaterials, Zusammensetzung der Emulsion, Sprührate der Emulsion (ca. 30 - ca. 80 g/min., [z.B. im Falle der Apparatur des Beispiels 1] bzw. 3-8 g/min*kg (Gesamtansatz), Struktur der rotierenden Bodenplatte - z.B. glatt, genoppt oder geriffelt, Drehzahl der rotierenden Bodenplatte, Zuluftgeschwindigkeit, Lufttemperatur (ca. 20 - ca. 80°C). Diese Beeinflussung durch die Art und Weise der Granulierung ist dem Fachmann bekannt und die enge Verteilung kann durch Versuche ermittelt werden. Wie eingangs erwähnt, führt sie im Falle des vorliegenden Verfahrens zu besonders günstigen Werten.

Unter "einem im wesentlichen staubfreien Produkt" wird im vorliegenden Fall ein Granulat verstanden, das einen Feinanteil (im wesentlichen Kernmaterial und Trägermaterial) aufweist, der unter 5% liegt, dies bei Partikelgrössen von <0,1 mm.

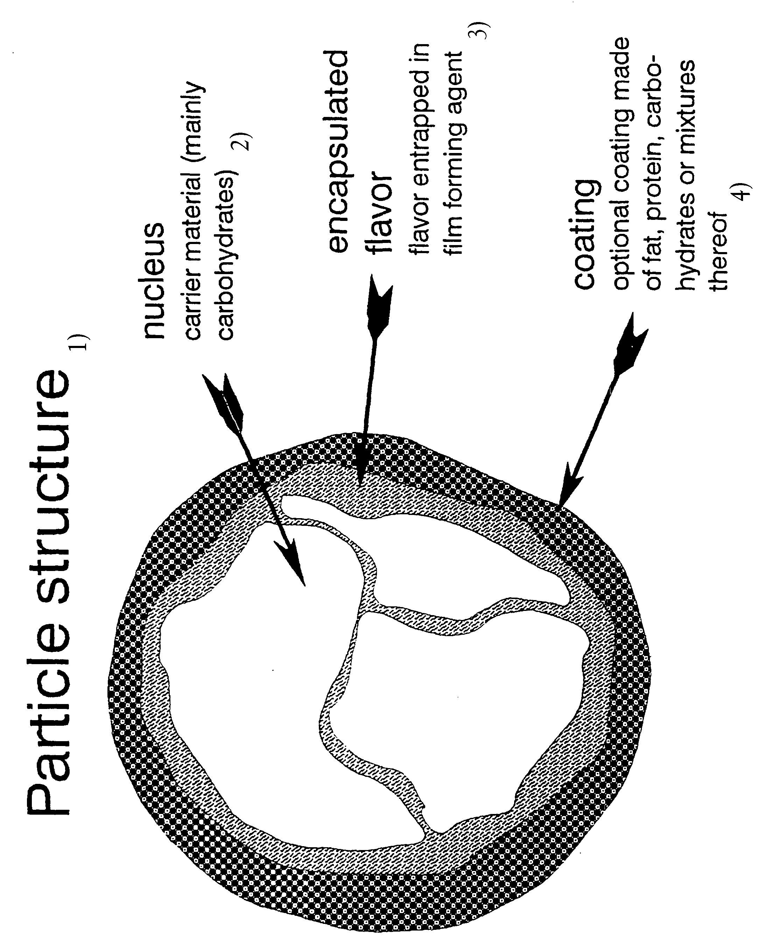

Ein erfindungsgemäss erhältliches, im wesentlichen sphärisches Granulat ist in der Figur 1 abgebildet.

### Beispiel 1: Zitronengranulat

Im Rotorgranulator (Typ GPCG-5, Firma Glatt, Pratteln) werden 2,975 kg Griesszucker und 2,975 kg Puderzucker vorgelegt.

In einem separaten Behälter werden 800 g Wasser bei Raumtemperatur vorgelegt, danach werden 595 g Maltodextrin und 105 g chemisch modifizierte Stärke (Dextrin) zugegeben und darin gelöst. Unter starkem Rühren (18500 UpM) mit einer Mischturbine vom Typ Polytron der Firma Kinematika, Littau werden 350 g Zitronenaroma (praktisch ausschliesslich Zitronenöl) langsam zugegeben. Es wird noch 3 Minuten weiter homogenisiert, bis eine stabile Sprühemulsion entsteht. Der Rotorgranulator wird in Betrieb gesetzt und die Sprühemulsion (70 g/min.) über eine Zweistoffdüse (Emulsion/Druckluft) submers, ca. auf der halben Höhe des Fliessbetts zugepumpt. Wenn die Sprühemulsion aufgebraucht ist, wird das Aromengranulat noch während 5-10 Minuten nachgetrocknet. Dadurch entsteht ein freifliessendes Aromengranulat mit einer Korngrössenverteilung von 87% innerhalb 0,2-1,0 mm und einem Schüttgewicht von 0,65 g/ml.

### Beispiel 2: Limettegranulat

Als Kernmaterial werden 4,662 kg Isomalt vorgelegt.

Die Sprühemulsion wird wie im Beispiel 1 hergestellt und besteht aus 2 1 Wasser, 1,260 kg Gummi arabicum, 26,6 g Tartrazin (gelber Farbstoff), 1,4 g Indigotin (blauer Farbstoff) und 1,050 kg Limettenaroma (praktisch ausschliesslich Limettenöl). Das Kernmaterial wird in Bewegung versetzt (350 U/min.) bzw. fluidisiert und die Sprühemulsion mit 75 g/min. eingesprüht. Es entsteht ein Aromengranulat mit 87% der Partikeln zwischen 0,2 und 0,8 mm und einem Schüttgewicht von 0.71 g/ml.

### Beispiel 3: Gewürzgranulat

Die Vorlage setzt sich aus 1,680 kg Kochsalz, 1,680 kg Kristallzucker und 2,380 kg HVP-Pulver (hydrolysiertes Pflanzenprotein) zusammen.

Wie im Beispiel 1 wird die Sprühemulsion hergestellt, welche 1 l Wasser, 679,7 g Maltodextrin, 210 g modifizierte Stärke und 370,3 g einer Gewürz-Aromenkomposition (basierend auf Zitronengrasöl + Pfefferoleoresin) enthält. Durch das Starten des Rotorgranulators (300 U/min.) wird die Vorlage gemischt und in Bewegung versetzt. Die Sprühemulsion wird eingesprüht (30 g/min.) Sobald fertig granuliert und getrocknet ist, werden 350 g eines geschmolzenen Pflanzenfettes mit einer Temperatur von 50-60°C eingesprüht. Die Temperatur im Granulator ist zu diesem Zeitpunkt unter 40°C, womit das Fett auf der Granulatoberfläche erstarrt. Dadurch wird ein umhülltes Granulat mit Gewürzgeschmack erhalten.

### Beispiel 4: Fruchtaroma

In diesem Fall werden 2,450 kg Griesszucker und 3,500 kg eines Multivitaminpräparates (Hoffmann-La Roche, Basel) im Rotorgranulator vorgelegt. Die Sprühemulsion setzt sich aus 1 l Wasser, 595 g Maltodextrin, 105 g modifizierter Stärke und 350 g Fruchtaroma zusammen und wird analog zum Beispiel 1 hergestellt. Der Granulator wird wie im Beispiel 1 betrieben, die Sprühemulsion zu 50 g/min. zudosiert.

### Beispiel 5: Parfümiertes Granulat

Im Rotorgranulator werden 5,250 kg Maltodextrin vorgelegt.

Die Sprühemulsion wird wie in den Beispielen 1 bis 4 hergestellt. Sie besteht aus 1,500 kg Wasser, 0,875 kg Maltodextrin. 0,175 g chemisch modifizierter Stärke und 0,700 kg eines beliebigen Riechstoffgemisches für Parfümeriezwecke.

Das Kernmaterial wird in Bewegung versetzt (400 U/min.) bzw. fluidisiert und die Sprühemulsion mit 85 g/min. eingespritzt. Es entsteht ein Parfümeriegranulat, das mit 0,350 kg Polyglykol 6000S, gelöst in 0,350 kg Ethanol, und 0,175 kg Wasser umhüllt wird. Das Wasser und das Ethanol verdampfen und das Polyglykol bildet einen Film um das Riechstoffgranulat.

Die im Rahmen der vorliegenden Erfindung angegebenen Apparateparameter beziehen sich immer auf den für den Laborbetrieb geeigneten Rotorgranulator GPCG-5 mit einem Durchmesser von 50 cm und einer Höhe von 1,9 m (Fliessbett 92 cm); für den Fall anderer Apparatedimensionen sind Abweichungen möglich.

Die in den Beispielen 1 bis 5 erwähnten Aromen- und Riechstoffgranulate werden in den zu aromatisierenden Lebensmitteln, wie z.B. Teepulver, Kräutermischungen, Kaugummi, gefrorenen Fertigmenues, Weich- und Hartbonbons, Biskuit, Eiscrème, Eiscremecoating, Schokoladeriegeln, Constant-Getränkepulvern, Suppen- und Saucenbeuteln, Mundhygieneprodukten, wie Gebissreinigungstabletten und Zahnpasten, etc. eingesetzt oder in den zu parfümierenden Kosmetik-, Hygiene-, Pharma-, Seifen-, Detergenzien- oder Haushaltprodukten verwendet.

### Beispiel 6: Applikationen für die erfindungsgemässen Granulate

### Teebeutel

Zu gebrochenen Teeblättern wird 2-12% granuliertes Aroma, z.B. Limette, gemischt und in Teebeutel verpackt.

Die Vorteile gegenüber konventionellen Pulveraromen sind die folgenden:
- Das Granulat dringt aufgrund der Partikelgrösse nicht durch die Papierporen => kein Verlust bei Transport und Lagerung.
- Kein Abrieb der Granulatpartikel bei Verarbeitung und Transport, da mechanisch resistent.
- Das Aroma löst sich rasch und vollständig auf, wenn der Teebeutel in heisses Wasser getaucht wird.

### Instant Getränkpulver

Zu einem Instant Getränkpulver auf Zuckerbasis wird 1-2% eines granulierten Aromas, z.B. Tropenfrucht-Mix, gemischt.

Die Vorteile gegenüber konventionellen Pulveraromen sind insbesondere:
- Keine Entmischung bei Abpackung und Transport, da die Granulatpartikelgrösse auf die Partikelgrösse des Getränkpulvers abgestimmt wird.
- Verbesserte Lagerstabilität der erfindungsgemässen Citrusaromen.
- Schnelle Auflösung des Aromas, wenn das Getränkpulver in kaltem Wasser gerührt wird.

### Kaugummi

Ein orange-gefärbtes granuliertes Aroma, z.B. Pfirsich wird zu 0,5% in eine Kaugummimasse eingearbeitet, die bereits ein flüssiges Aroma, z.B. Zitrone enthält.

Der Kaugummi weist dadurch die folgenden Vorteile auf, die mit konventionellen Pulveraromen nicht erreicht werden können:
- attraktive, gut sichtbare Partikel, die während der Lagerung stabil bleiben.
- ein doppelter Aroma-Effekt, wobei die beiden Aromen separat wahrgenommen werden.
- ein sehr rasches Wahrnehmen des granulierten Aromas nach wenigen Sekunden Kauens, gefolgt vom flüssigen Aroma. Bei jedem Biss auf ein Granulat-Korn wird Aroma wieder neu freigesetzt.

### Hart- und Kaubonbons

Ein braun gefärbtes granuliertes Aroma, z.B. Zimt, wird zu 0,2-0,4% in eine Hart- oder Kaubonbonmasse eingemischt, die mit einem flüssigen Aroma, z.B. Apfel aromatisiert ist.

### Vorteile:

- attraktive, sichtbare Partikel, die während der Lagerung stabil bleiben.
- ein doppelter Aroma-Effekt, wobei die beiden Aromen separat wahrgenommen werden. In diesem Fall entsteht der Eindruck von Apfelstrudel.

### Schokolade

Ein Cola-Aroma, das Na-bicarbonat und Zitronensäure enthält, wird zu 1-4% in eine Schokoladenmasse eingearbeitet.

### Vorteil:

Beim Essen entwickelt sich sofort ein prickelnder Effekt, indem sich unter dem Einfluss der Speichelfeuchtigkeit Kohlensäure bildet.

### Icecream mit Schokoladeüberzug

In die Schokolademasse wird 1-3% eines granulierten Fruchtaromas, z.B. Zitrone, das auch Zitronensäure enthält, eingemischt. Die Schokolade wird auf die übliche Art als dünner Ueberzug auf das fertige Icecream aufgetragen.

Beim Konsumieren des Icecreams machen sich folgende Vorteile bemerkbar:
- Oertlich definierter fruchtiger Geschmack, wie von Fruchtpartikeln, in der Schokolade.
- Der Fruchteindruck wird verstärkt durch die Zitronensäure, was mit üblichen Pulveraromen nicht möglich ist.
- Granulierte Aromen mit einer Partikelgrösse von 0,8-2 mm ergeben zusätzlich einen knusprigen Effekt beim Daraufbeissen.

### Gefrorenes Reisgericht

Ein Würzaroma, das Salz enthält und zusätzlich mit einer Hartfettschicht überzogen ist (Beispiel 3), wird zum gekochten, abgekühlten Reis gemischt oder aufgestreut.

### Vorteil:

Da das Salz in den Partikeln eingeschlossen ist, löst es sich nicht auf und verursacht keine Aenderung des Gefrierverhaltens des Reisgerichtes.

### Legende

1) Teilchenstruktur
2) Nukleus, Kern
   (Trägermaterial, hauptsächlich Kohlehydrate)
3) enkapsuliertes Aroma
   (Aroma ist im filmbildenden Agens eingeschlossen)
4) Umhüllung, Hülle
   (fakultativ, z.B. aus Fett, Protein, Kohlehydraten oder Gemischen davon)

## Patentansprüche

1. Verfahren zur Herstellung von sphärischem Granulat, **dadurch gekennzeichnet, dass** in ein in einem Fliessbett-rotorgranulator fluidisiertes Kernmaterial eine Aroma- bzw. Riechstoffemulsion submers eingesprüht und auf diese Weise granuliert wird.

2. Verfahren nach Anspruch 1, worin das Kernmaterial ein in der Lebensmittel-, der kosmetischen, pharmazeutischen oder Konsumgüterindustrie zugelassener Feststoff ist und zweckmässigerweise Korngrössen von ca. 0,02 bis ca. 3,0 mm aufweist.

3. Verfahren nach Anspruch 1 oder 2, **dadurch gekennzeichnet, dass** das Kernmaterial ein Kohlenhydrat, z.B. ein Zucker wie Glucose, Lactose, Saccharose, Stärke oder abgebaute Stärke, oder ein Zuckeralkohol wie Isomalt, oder Pectin, hydrolysiertes Pflanzenprotein, Nahrungsfasern, organische oder anorganische Salze darstellt.

4. Verfahren nach Anspruch 1, 2 oder 3, worin das Trägermaterial für die Sprühemulsion chemisch modifizierte Stärke, abgebaute Stärke, wie z.B. Maltodextrine, natürliche Harze, Exudate wie z.B. Gummi arabicum, Gelatine oder Pflanzenextrakte wie z.B. Carragenan oder Alginate, in Wasser oder Wasser/Alkohol-Gemischen ist.

5. Verfahren nach Anspruch 1 bis 4, worin bei der Herstellung der Sprühemulsion eine individuelle Aromen- oder Riechstoffkomponente oder ein Gemisch von verschiedenen Komponenten natürlicher oder synthetischer Herkunft eingesetzt wird.

6. Verfahren nach Anspruch 1 bis 5, worin der Aromen- oder Riechstoffanteil ca. 1 bis ca. 25%, insbesondere ca. 5 bis ca. 15%, vorzugsweise ca. ≤10%, des fertigen Aromen- oder Riechstoffgranulates ausmacht und der Rest im wesentlichen aus Kernmaterial und Trägermaterial besteht.

7. Verfahren nach Anspruch 1 bis 6, worin die an der Erzeugung des Fliessbetts mitbeteiligte Luft auf ca. 20 bis ca. 80°C, insbesondere auf ca. 40 bis ca. 60°C, erwärmt wird.

8. Verfahren nach Anspruch 1 bis 7, worin ein Aromen- oder Riechstoffgranulat mit einer Partikelgrösse im Bereich von ca. 0.05 bis ca. 3,0, vorzugsweise im Bereich von ca. 0,2 bis ca. 1,5 mm hergestellt wird.

9. Verfahren nach Anspruch 1 bis 8, worin ein Produkt hergestellt wird, das zu ≥95% aus Partikeln von ca. 0,2 - ca. 3 mm Durchmesser, bzw. zu ca. 80% aus Partikeln, die eine Bandbreite von nur ca. 0,6 mm aufweisen, besteht.

10. Verfahren nach Anspruch 1 bis 9, worin nach der Granulierung eine Umhüllung der Partikel, erfolgt, und zwar durch submerses Aufsprühen einer Lösung, Emulsion oder Schmelze einer Substanz bzw. eines Substanzgemisches, wie z.B. Fett, oder modifizierter Cellulose, Gelatine, Pflanzen- oder Tierextrakten, Exudaten wie Pflanzengummi, abgebaute Stärke oder chemisch modifizierte Stärke, pharmazeutisch anwendbaren Kunststoffen, etc.

11. Verfahren nach Anspruch 1 bis 10, worin bei der Granulierung Zusatzstoffe wie künstliche Süssstoffe, Lebensmittelfarbstoffe, Vitamine, Antioxidantien, Antischaummittel, Kohlensäuregeneratoren, Genusssäuren verwendet werden, die als solche dem Kernmaterial oder der Sprühemulsion zugesetzt werden.

12. Verwendung eines Fliessbett-rotorgranulators bei der Herstellung von sphärischem Aromen- oder Riechstoffgranulat.

13. Verwendung eines gemäss Verfahren einer der Ansprüche 1 bis 11 hergestellten Aromen- oder Riechstoffgranulats zu Aromatisierungs- oder Parfümierungszwecken.

14. Verfahren zur Herstellung von sphärischem Aromengranulat gemäss Anspruch 1, **dadurch gekennzeichnet, dass** in ein in einem Fliessbett-rotorgranulator fluidisiertes Kernmaterial eine Aromaemulsion submers eingesprüht und auf diese Weise granuliert wird.

15. Verfahren nach Anspruch 14, worin das Kernmaterial ein in der Lebensmittelindustrie zugelassener Feststoff ist und zweckmässigerweise Korngrössen von ca. 0,02 bis ca. 3,0 mm aufweist.

16. Verfahren nach Anspruch 14 oder 15, **dadurch gekennzeichnet, dass** das Kernmaterial ein Kohlenhydrat, z.B. ein Zucker wie Glucose, Lactose, Saccharose, oder ein Zuckeralkohol wie Isomalt, oder Pectin, hydrolysiertes Pflanzenprotein, Nahrungsfasern, organische oder anorganische Salze darstellt.

17. Verfahren nach Anspruch 14, 15 oder 16, worin das Trägermaterial für die Sprühemulsion chemisch modifizierte Stärke, abgebaute Stärke, wie z.B. Maltodextrinen, natürliche Harze, Exudate wie z.B. Gummi arabicum, Gelatine oder Pflanzenextrakte wie z.B. Carragenan oder Alginaten, in Wasser oder Wasser/Alkohol-Gemischen ist.

18. Verfahren nach Anspruch 14 bis 17, worin bei der Herstellung der Sprühemulsion eine individuelle Aromenkomponente oder ein Gemisch von verschiedenen Komponenten natürlicher oder synthetischer Herkunft eingesetzt wird.

19. Verfahren nach Anspruch 14 bis 18, worin der Aromenanteil ca. 1 bis ca. 25%, insbesondere ca. 5 bis ca. 15% des fertigen Aromengranulates ausmacht und der Rest im wesentlichen aus Kernmaterial und Trägermaterial besteht.

20. Verfahren nach Anspruch 14 bis 19, worin die an der Erzeugung des Fliessbetts mitbeteiligte Luft auf ca. 20 bis ca. 80°C, insbesondere auf ca. 40 bis ca. 60°C, erwärmt wird.

21. Verfahren nach Anspruch 14 bis 20, worin ein Aromengranulat mit einer Partikelgrösse im Bereich von ca. 0.05 bis ca. 3,0, vorzugsweise im Bereich von ca. 0,2 bis ca. 1,5 mm hergestellt wird.

22. Verfahren nach Anspruch 14 bis 21, worin ein Produkt hergestellt wird, das zu ≥95% aus Partikeln von ca. 0,2 - ca. 3 mm Durchmesser, bzw. zu ca. 80% aus Partikeln, die eine Bandbreite von nur ca. 0,6 mm aufweisen, besteht.

23. Verfahren nach Anspruch 14 bis 22, worin nach der Granulierung eine Umhüllung der Partikel, erfolgt, und zwar durch Aufsprühen einer Lösung, Emulsion oder Schmelze einer Substanz bzw. eines Substanzgemisches, wie z.B. Fett, oder modifizierter Cellulose, Gelatine, Pflanzenoder Tierextrakten, Exudaten wie Pflanzengummi, abgebaute Stärke oder chemisch modifizierte Stärke, pharmazeutisch anwendbaren Kunststoffen, etc.

24. Verfahren nach Anspruch 14 bis 23, worin bei der Granulierung Zusatzstoffe wie künstliche Süssstoffe, Lebensmittelfarbstoffe, Vitamine, Antioxidantien, Antischaummittel, Kohlensäuregeneratoren, Genusssäuren verwendet werden, die als solche dem Kernmaterial oder der Sprühemulsion zugesetzt werden.

25. Verwendung eines Fliessbett-rotorgranulators bei der Herstellung von sphärischem Aromengranulat.

26. Verwendung eines gemäss Verfahren einer der Ansprüche 14 bis 24 hergestellten Aromengranulats zu Aromatisierungszwecken.

## Claims

1. A method of producing a spherical granulate, **characterised in that** a flavour or perfume emulsion is sprayed under the surface of a core material fluidised in a fluidised-bed rotor granulator and thus granulated.

2. A method according to claim 1, wherein the core material is a solid permitted in the food, cosmetics, pharmaceutical or consumer-goods industry and advantageously has a particle size from about 0.02 to about 3.0 mm.

3. A method according to claim 1 or 2, **characterised in that** the core material is a carbohydrate, e.g. a sugar such as glucose, lactose, saccharose, starch or degraded starch or a sugar alcohol such as isomaltose, pectin, hydrolysed vegetable protein, food fibres, organic or inorganic salts.

4. A method according to claim 1, 2 or 3, wherein the carrier material for the spray emulsion is chemically modified starch, degraded starch, e.g. maltodextrins, natural resins, exudates such as gum arabic, gelatine or vegetable extracts such as carragheenin or alginates in water or water/alcohol mixtures.

5. A method according to claims 1 to 4, wherein an individual flavour or perfume component or a mixture of different components of natural or synthetic origin is used in the production of the spray emulsion.

6. A method according to claims 1 to 5, wherein the flavour or perfume component makes up about 1 to about 25%, particularly about 5 to about 15%, preferably up to about 10%, of the finished flavour or perfume granulate and the remainder consists substantially of core material and carrier material.

7. A method according to claims 1 to 6, wherein the air for generating the fluidised bed is heated to about 20 - 80°C, more particularly to about 40 - 60°C.

8. A method according to claims 1 to 7, wherein a flavour or perfume granulate is produced with a particle size in the range from about 0.05 to about 3.0 mm, preferably in the range from about 0.2 to about 1.5 mm.

9. A method according to claims 1 to 8, wherein a substance is produced comprising up to 95% particles about 0.2 - 3 mm in diameter or about 80% particles in a band width of only 0.6 mm.

10. A method according to claims 1 to 9, wherein the particles are coated after granulation, by under-surface spraying of a solution, emulsion or melted substance or mixture of substances such as fat, modified cellulose, gelatine, vegetable or animal extracts, exudates such as gums, degraded starch or chemically modified starch, plastics for use in pharmaceuticals, etc.

11. A method according to claims 1 to 10, wherein substances are added during granulation, e.g. artificial sweeteners, food dyes, vitamins, anti-oxidising agents, anti-foaming agents, carbon dioxide generators or luxury food acids, added as such to the core material or the spray emulsion.

12. Use of a fluidised-bed rotor granulator in the production of spherical flavour or perfume granulate.

13. Use of a flavour or perfume granulate produced by a method according to any of claims 1 to 11 for flavouring or perfuming.

14. A method of using spherical flavour granulates according to claim 1, **characterised in that** a flavour emulsion is sprayed under the surface into a core material fluidised in a fluidised-bed rotor granulator and thus granulated.

15. A method according to claim 14, wherein the core material is a solid permitted in the food industry and advantageously has a particle size of about 0.02 to about 3.0 mm.

16. A method according to claim 14 or 15, **characterised in that** the core material is a carbohydrate, e.g. a sugar such as glucose, lactose or saccharose or a sugar alcohol such as isomaltose or pectin, hydrolysed vegetable protein, food fibres or organic or inorganic acids.

17. A method according to claim 14, 15 or 16, wherein the carrier material for the spray emulsion is chemically modified starch, degraded starch such as maltodextrins, natural resins, exudates such as gum arabic, gelatine or plant extracts such as carragheenin or alginates, in water or water/alcohol mixtures.

18. A method according to claims 14 to 17, wherein an individual flavour component or a mixture of different components of natural or synthetic origin is used in the production of the spray emulsion.

19. A method according to claims 14 to 18, wherein the flavour makes up about 1 to about 25%, particularly about 5 to about 15% of the finished flavour granulate and the remainder consists mainly of core material and carrier material.

20. A method according to claims 14 to 19, wherein the air for generating the fluidised bed is heated to about 20 - 80°C, particularly to about 40 - 60°C.

21. A method according to claims 14 to 20, wherein a flavour granulate is produced with a particle size in the range from about 0.05 to about 3.0 mm, preferably in the range from about 0.2 to about 1.5 mm.

22. A method according to claims 14 to 21, wherein a substance is produced consisting of up to 95% particles about 0.2 - 3 mm in diameter or about 80 % of particles having a band width of only about 0.6 mm.

23. A method according to claims 14 to 22, wherein the particles are coated after granulation by spraying a solution, emulsion or melted substance or mixture of substances such as fat, modified cellulose, gelatine, plant or animal extracts, exudates such as gum, degraded starch or chemically modified starch, or plastics of use in pharmaceuticals.

24. A method according to claims 14 to 23, wherein substances such as artificial sweeteners, food dyes, vitamins, anti-oxidising agents, anti-foaming agents, carbon dioxide generators or luxury-food acids are used in the granulation process and added as such to the core material for the spray emulsion.

25. Use of a fluidised-bed rotor granulator for production of spherical flavour granulate.

26. Use of a flavour granulate produced by the method according to any of claims 14 to 24 for flavouring purposes.

## Revendications

1. Procédé pour la préparation d'un granulat sphérique, **caractérisé en ce qu'**une émulsion d'arôme ou de parfum est pulvérisée de manière submersible dans un matériau formant le noyau qui est fluidisé dans un granulateur rotatif à lit fluidisé et qu'elle est granulée de cette manière.

2. Procédé selon la revendication 1, dans lequel le matériau formant le noyau est un solide admis dans l'industrie alimentaire, cosmétique, pharmaceutique ou des biens de consommation et présente opportunément des grosseurs de granule d'environ 0,02 à environ 3,0 mm.

3. Procédé selon la revendication selon la revendication 1 ou 2, **caractérisé en ce que** le matériau formant le noyau est un hydrate de carbone, par exemple un sucre tel que du glucose, du lactose, du saccharose, de l'amidon ou de l'amidon dégradé, ou un alcool de sucre, tel que de l'isomalt, ou de la pectine, une protéine végétale hydrolysée, des fibres alimentaires, des sels organiques ou inorganiques.

4. Procédé selon la revendication 1, 2 ou 3, dans lequel le matériau support pour l'émulsion pulvérisée est de l'amidon modifié chimiquement, de l'amidon dégradé, tel que par exemple de la maltodextrine, des résines naturelles, des exsudats, tels que par exemple de la gomme arabique, de la gélatine ou des extraits végétaux, tels que par exemple du carragène ou des alginates, dans de l'eau ou des mélanges eau/alcool.

5. Procédé selon la revendication 1 à 4, dans lequel on utilise lors de la préparation de l'émulsion pulvérisée un composant individuel d'arôme ou de parfum ou un mélange de différents composants d'origine naturelle ou synthétique.

6. Procédé selon la revendication 1 à 5, dans lequel la proportion d'arôme ou de parfum représente environ 1 à environ 25 %, en particulier environ 5 à environ 15 %, de préférence environ ≤ 10 % du granulat d'arôme ou de parfum fini et le reste est essentiellement constitué de matériau formant le noyau et de matériau support.

7. Procédé selon la revendication 1 à 6, dans lequel l'air participant à la réalisation du lit fluidisé est chauffé à environ 20 jusqu'environ 80°C, en particulier à environ 40 jusqu'environ 60°C.

8. Procédé selon la revendication 1 à 7, dans lequel on prépare un granulat d'arôme ou de parfum présentant une grosseur de particule dans la plage d'environ 0,05 à environ 3,0, de préférence dans la plage d'environ 0,2 à environ 1,5 mm.

9. Procédé selon la revendication 1 à 8, dans lequel on prépare un produit qui est constitué de jusqu'à ≥ 95 % de particules d'environ 0,2 à environ 3 mm de diamètre ou de jusqu'environ 80 % de particules qui présentent une largeur de bande de seulement 0,6 mm.

10. Procédé selon la revendication 1 à 9, dans lequel on réalise après la granulation un enrobage des particules et ce par une pulvérisation submersible d'une solution, d'une émulsion ou d'une masse fondue d'une substance ou d'un mélange de substances, tels que par exemple de la graisse ou de la cellulose modifiée, de la gélatine, des extraits végétaux ou animaux, des exaudats tels que de la gomme végétale, de l'amidon dégradé ou de l'amidon chimiquement modifié, des matériaux synthétiques pouvant être utilisés en pharmacie, etc.

11. Procédé selon la revendication 1 à 10, dans lequel on utilise lors de la granulation des additifs tels que des édulcorants synthétiques, des colorants alimentaires, des vitamines, des antioxydants, des antimousses, des générateurs d'acide carbonique, des acides de consommation, qui sont ajoutés tels quels au matériau formant le noyau ou à l'émulsion pulvérisée.

12. Utilisation d'un granulateur rotatif à lit fluidisé lors de la préparation d'un granulat d'arôme ou de parfum sphérique.

13. Utilisation d'un granulat d'arôme ou de parfum préparé selon le procédé de l'une quelconque des revendications 1 à 11 à des fins d'aromatisation ou pour donner un parfum.

14. Procédé pour la préparation d'un granulat d'arôme sphérique selon la revendication 1, **caractérisé en ce qu'**une émulsion d'arôme est pulvérisée de manière submersible dans un matériau formant le noyau qui est fluidisé dans un granulateur rotatif à lit fluidisé et qu'elle est granulée de cette manière.

15. Procédé selon la revendication 14, dans lequel le matériau formant le noyau est un solide admis dans l'industrie alimentaire et présente opportunément des grosseurs de granule d'environ 0,02 à environ 3,0 mm.

16. Procédé selon la revendication 14 ou 15, **caractérisé en ce que** le matériau formant le noyau est un hydrate de carbone, par exemple un sucre tel que du glucose, du lactose, du saccharose, ou un alcool de sucre, tel que de l'isomalt, ou de la pectine, une protéine végétale hydrolysée, des fibres alimentaires, des sels organiques ou inorganiques.

17. Procédé selon la revendication 14, 15 ou 16, dans lequel le matériau support pour l'émulsion pulvérisée est de l'amidon modifié chimiquement, de l'amidon dégradé, tel que par exemple des maltodextrines, des résines naturelles, des exsudats, tels que par exemple de la gomme arabique, de la gélatine ou des extraits végétaux, tels que par exemple du carragène ou des alginates, dans de l'eau ou des mélanges eau/alcool.

18. Procédé selon la revendication 14 à 17, dans lequel on utilise lors de la préparation de l'émulsion pulvérisée un composant individuel d'arôme ou un mélange de différents composants d'origine naturelle ou synthétique.

19. Procédé selon la revendication 14 à 18, dans lequel la proportion d'arôme représente environ 1 à environ 25 %, en particulier environ 5 à environ 15 % du granulat d'arôme fini et le reste est essentiellement constitué de matériau formant le noyau et de matériau support.

20. Procédé selon la revendication 14 à 19, dans lequel l'air participant à la réalisation du lit fluidisé est chauffé à environ 20 jusqu'environ 80°C, en particulier à environ 40 jusqu'environ 60°C.

21. Procédé selon la revendication 14 à 20, dans lequel on prépare un granulat d'arôme présentant une grosseur de particule dans la plage d'environ 0,05 à environ 3,0, de préférence dans la plage d'environ 0,2 à environ 1,5 mm.

22. Procédé selon la revendication 14 à 21, dans lequel on prépare un produit qui est constitué de jusqu'à ≥ 95 % de particules d'environ 0,2 à environ 3 mm de diamètre ou de jusqu'environ 80 % de particules qui présentent une largeur de bande de seulement 0,6 mm.

23. Procédé selon la revendication 14 à 22, dans lequel on réalise après la granulation un enrobage des particules et ce par une pulvérisation d'une solution, d'une émulsion ou d'une masse fondue d'une substance ou d'un mélange de substances, tels que par exemple de la graisse ou de la cellulose modifiée, de la gélatine, des extraits végétaux ou animaux, des exsudats tels que de la gomme végétale, de l'amidon dégradé ou de l'amidon chimiquement modifié, des matériaux synthétiques pouvant être utilisés en pharmacie, etc.

24. Procédé selon la revendication 14 à 23, dans lequel on utilise lors de la granulation des additifs tels que des édulcorants synthétiques, des colorants alimentaires, des vitamines, des antioxydants, des antimousses, des générateurs d'acide carbonique, des acides de consommation, qui sont ajoutés tels quels au matériau formant le noyau ou à l'émulsion pulvérisée.

25. Utilisation d'un granulateur rotatif à lit fluidisé lors de la préparation d'un granulat d'arôme ou de parfum sphérique.

26. Utilisation d'un granulat d'arôme préparé selon le procédé de l'une quelconque des revendications 14 à 24 à des fins d'aromatisation.
